# EUROPEAN PATENT APPLICATION

(11) **EP 3 187 106 A1**
(43) Date of publication of application: **05.07.2017**
(21) Application number: 14900906.0
(22) Date of filing: 06.11.2014
(51) Int. Cl.: A61B 5/021

(54) **BLOOD PRESSURE MEASURING AUXILIARY DEVICE, BLOOD PRESSURE MEASURING DEVICE, AND DESIGN METHOD THEREFOR**

(30) Priority: 29.08.2014 CN 201410436105
(71) Applicant: Zhou, Jialu, Shanghai 200080 (CN)
(72) Inventor: Zhou, Jialu, Shanghai 200080 (CN)
(74) Representative: Stuttard, Garry Philip
(86) International application number: PCT/CN2014/090433
(87) International publication number: WO 2016/029546

(57) **Abstract**

A blood pressure measuring auxiliary device, a blood pressure measuring equipment and a design method therefor are provided. During a blood pressure measurement, the measured pressure values and beating sound are recorded, so that the recorded pressure values and sound may be played back, which improves the accuracy of the auscultatory method; and the parameters used in the oscillometric method may be adjusted, which improves the accuracy of the oscillometric method. The blood pressure measuring auxiliary device is arranged so that it can be connected with a pressuring device of a sphygmomanometer (5), and comprises a sensing module (1), a communication module, and an audio collection module (2). The sensing module (1) is used for sensing the pressure at the joint between the blood pressure measuring auxiliary device and the pressuring device and outputting a pressure signal to the communication module. The communication module is used for receiving the pressure signal to be transmitted thereby to a terminal equipment (6). The audio collection module (2) is used for collecting the sound at a measured site and outputting an audio signal to the terminal equipment (6).

## Description

### Field of the Invention

The present invention relates to the field of blood pressure monitoring and, in particular, to a blood pressure measuring auxiliary device, a blood pressure measuring equipment and a design method therefor.

### Description of the Prior art

The sphygmomanometers currently on the market are primarily based on two measurement methods: the auscultatory method and the oscillometric method.

The auscultatory method is the main method used in clinical medical treatment. The mercury sphygmomanometer used by physicians is designed with the auscultatory method. With the auscultatory method, the blood pressure values are determined by means of listening to the sounds of the brachial artery blood flow with a stethoscope during deflation of the cuffed air bladder of the sphygmomanometer in combination with the corresponding pressure. The scale indicated by the mercury column is a systolic pressure at the moment when the first beating sound occurs in the stethoscope; the scale indicated by the mercury column is a diastolic pressure at the moment when the beating sounds suddenly become weaker or disappear. The auscultatory method, due to its high measurement accuracy, is medically referred to as the golden standard of blood pressure measurement. In general, the studies for measurement accuracy of other types of sphygmomanometers are all carried out through comparing with the auscultatory method using the mercury sphygmomanometer. But the auscultatory method also has significant limitations in the use of an electronic sphygmomanometer, mainly because the electronic equipment's ability to discriminate the sounds is weaker than that of human ears in some aspects. The auscultatory method needs to determine the strength (height of beating sound) and frequency (being beating sound or noise) of sounds, the electronic sphygmomanometer using the auscultatory method is less popularized due to the design difficulty and cost, and the ability thereof to resist noise interference is poor. The mercury sphygmomanometer using the auscultatory method (or other non-mercury media) is also inconvenient to use, for example, it is difficult for one to measure on oneself, where specialized training is required; the ears and eyes need to be highly synchronized when making a measurement so as to capture the variations of the sounds and the corresponding pressure values, where the data is not recorded automatically.

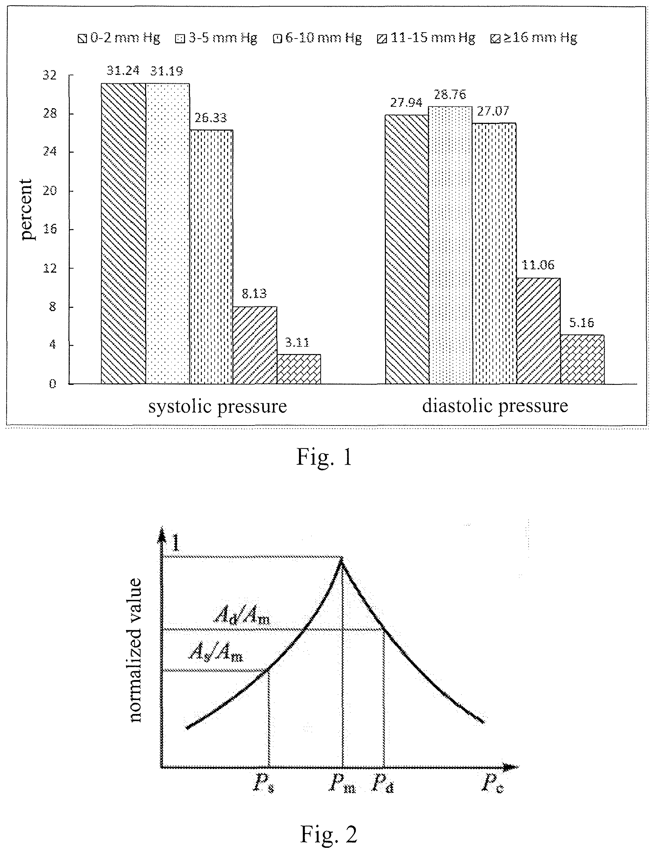

The oscillometric method determines the blood pressure values by measuring the amplitude of the pulse oscillation wave in combination with the corresponding cuff pressure. Since the principle is realized simply, the electronic sphygmomanometers which measure the blood pressure using the oscillometric method are the mainstream products on the market, for example, most of the electronic sphygmomanometers of Omron are all using the oscillometric method. Many studies found, by comparison, that the accuracy of the oscillometric method is very poor sometimes. The detection report for one mainstream brand sphygmomanometer products made by America's Centers for Disease Control shows that, the measurement deviation of that brand's one type of sphygmomanometers may be up to 16mmHg higher than the mercury sphygmomanometer, as shown in Figure 1, which is the diagram of the frequency distribution of the measurement deviations of this brand's sphygmomanometer. One source of inaccuracy of the oscillometric method may be associated with the measuring principle itself.

In the auscultatory method, during deflation of the cuffed air bladder, the blood cannot flow before the pressure drops to the systolic pressure, so human ears can't hear the beating sound of blood flow. In the oscillometric method, the oscillation wave still exists before the blood pressure drops to the systolic pressure, but the amplitude thereof is smaller. During deflation of the cuff or the dropping of air bladder pressure, the amplitude of variation of the oscillation wave is continuous before and after the systolic pressure and the diastolic pressure. This is not like that, in the auscultatory method, there is a fine limit which is the occurrence of the beating sound out of nothing. Therefore, the oscillometric method can only set up an empirical correlation between the oscillation amplitude of variation and the pressure values by a large number of individual specimens. The average correlation that is set up based on a large number of individual specimens is not suited for all individuals, which results in that for a certain proportion of people, the measurement errors of the blood pressure values are large. The errors are not caused by the quality of the sphygmomanometer itself, but the inherent defects of the measuring principle.

A common method used in the oscillometric method is the amplitude parameter method, also known as 'normalization method'. It normalizes the amplitude of the vibration signal of pulse wave with respect to the maximum amplitude of the signal, recognizing the systolic pressure and the diastolic pressure by determining the normalized parameters of the systolic pressure and the diastolic pressure, as shown in Figure 2. In this method, As is the amplitude of pulse wave corresponding to the systolic pressure, Am is the amplitude of the pulse wave corresponding to the mean pressure, Ad is the amplitude of pulse wave corresponding to the diastolic pressure, As/Am is the normalized value of the systolic pressure Pd, Ad/Am is the normalized value of the diastolic pressure Ps, Pc is the cuff pressure, and the abscissa represents a continuous reduction of the pressure inside the cuff during deflation. As/Am=C1, Ad/Am=C2, which correspond to the positions of the systolic pressure and the diastolic pressure, respectively. According to the measured amplitude of pulse wave and the corresponding static pressure, it is possible to obtain the systolic pressure Ps, the diastolic pressure Pd and the mean pressure Pm. In general, the amplitude parameter of the systolic pressure is 0.46 ∼ 0.64, the amplitude parameter of the diastolic pressure is 0.43 ∼ 0.73 (the principle of the oscillometric method may be referred to--Liu Jianqiang, Wang Yongcai. Electronic Sphygmomanometer system design Based On the Oscillometric Method [J]. Single-Chip Microcomputer and Embedded System Application, 2010(4):62-65.). Different sphygmomanometer manufacturers would use different amplitude parameters, which also results in that even if different manufacturers use identical oscillometric method, the measured values thereof are likely very different. The amplitude parameter corresponding to each measured individual may be different, and the sphygmomanometer does not use the unused parameter for everyone.

On the other hand, all the current sphygmomanometers designed by using the two methods collect data from themselves, through calculation, only informing the user about the results by displaying or by voice. The blood pressure values are determined during a measurement or directly by the sphygmomanometer, or the blood pressure values directly determined by human ears and eyes, rather than displaying the initial data to assist the user in determining the blood pressure values. And the visualization of the initial data during the measurement could play a good assisted effect on determining the blood pressure values, which is advantageous for improving the accuracy for determination of the blood pressure values. And the saved initial data is beneficial for determining the blood pressure values repeatedly, and the trend analysis may be conducted on the blood pressure conditions of the user, but current sphygmomanometers usually can only record several recently measured blood pressure values, further analysis also cannot be made and trend graphs cannot be shown on the sphygmomanometer.

### Summary of the Invention

In view of the above-mentioned drawbacks of the prior art, the design concept of the present invention is that, the initial data during a blood pressure measurement is completely collected and stored, and is numerically and/or graphically displayed after corresponding processing, so as to help the user to determine the blood pressure values more accurately. For example, the blood pressure values and beating sound (Korotkoff sound) are recorded during the blood pressure measurement, so that the above sound recording is listened to repeatedly to improve the accuracy of the auscultatory method, and the blood pressure values determined with the auscultatory method (or the blood pressure values with higher accuracy obtained by other ways) may be combined to adjust the parameters used in the oscillometric method, thereby improving the accuracy of the oscillometric method.

On the other hand, the user cannot obtain the initial data when making the measurement using the existing sphygmomanometer products, but can instantaneously read the blood pressure values artificially, or the blood pressure values are automatically determined and displayed by the sphygmomanometer, where the initial data for determining the blood pressure values cannot be extracted for the user, so that the above design concept cannot be achieved. In this regard, to obtain the initial data for the existing sphygmomanometer products is also a technical problem to be solved by the present invention.

In order to achieve the above-described object, the present invention provides a measuring auxiliary device for blood pressure measurement, the main effect of which is collecting and outputting a corresponding pressure signal when making the measurement using the existing sphygmomanometer products (the pressure signal when making the measurement cannot be obtained directly from the existing sphygmomanometer products), thereby providing a necessary data source for data analysis.

The measuring auxiliary device is arranged so that it can be connected with a pressuring device of the sphygmomanometer, and includes a sensing module and a communication module. The pressuring device mentioned in this specification refers to a component that is used for providing pressure to the measured site and can perform pressurization and depressurization operations, such as a combination of air pump, release valve, air tube and cuffed air bladder in an electronic sphygmomanometer, or a combination of manual air charging ball, air tube and cuffed air bladder in a mercury sphygmomanometer.

The sensing module is used for sensing the pressure at the joint between the measuring auxiliary device and the pressuring device and outputting a pressure signal to the communication module;
the communication module is used for receiving the pressure signal which is then transmitted to a terminal equipment.

The terminal equipment mentioned in this specification refers to an equipment that can implement data transmission with an external equipment through a wired or wireless connection and can save the received data. Preferably, it is an equipment that base on the above also can perform data analyzing and processing operations for the received data, and further output the data or the analyzed or processed data (such as graph, sound and the like), e.g., intelligent equipment or wearable equipment such as mobile phone, computer, tablet computer, bracelet and wrist watch.

Further, the sensing module includes a pressure sensor via which the pressure at the joint between the measuring auxiliary device and the pressuring device is sensed.

Further, the measuring auxiliary device also includes an operation module used for pre-processing the pressure signal, such as analog to digital conversion, noise reduction, signal amplification and filtering, and for transmitting the pre-processed pressure signal to the communication module to be transmitted thereby to the terminal equipment.

Further, the operation module determines the blood pressure values by the oscillometric method according to the pressure signal, and transmits the blood pressure values to the communication module to be transmitted thereby to the terminal equipment.

The communication module is wiredly and wirelessly connected with the terminal.

The present invention also provides another measuring auxiliary device for blood pressure measurement, the main effects of which is collecting and outputting the corresponding pressure signal and beating sound when making the measurement using the existing sphygmomanometer products (the pressure signal and beating sound when making the measurement cannot be obtained directly from the existing sphygmomanometer products), thereby providing a necessary data source for data analysis.

The measuring auxiliary device is arranged so that it can be connected with a pressuring device of the sphygmomanometer, and includes a sensing module, a communication module and an audio collection module;
The sensing module is used for sensing the pressure at the joint between the measuring auxiliary device and the pressuring device and outputting a pressure signal to the communication module;
The communication module is used for receiving the pressure signal and transmitting the pressure signal to a terminal equipment;
The audio collection module is used for collecting the sound at the measured site and outputting an audio signal to the terminal equipment.

Further, the sensing module includes a pressure sensor via which the pressure at the joint between the measuring auxiliary device and the pressuring device is sensed.

Further, the measuring auxiliary device also includes an operation module for pre-processing the pressure signal, such as analog to digital conversion, noise reduction, signal amplification and filtering, and for transmitting the pre-processed pressure signal to the communication module to be transmitted thereby to the terminal equipment.

Further, the operation module determines the blood pressure values by the oscillometric method according to the pressure signal and transmits the blood pressure values to the communication module to be transmitted thereby to the terminal equipment.

Further, the audio collection module includes a conversion module for pre-processing the collected sound (such as analog to digital conversion, noise reduction, signal amplification and filtering).

Further, the audio collection module transmits the audio signal to the communication module to be transmitted thereby to the terminal equipment. In this case, the audio signal is indirectly transmitted via the communication module to the terminal equipment.

Further, the audio collection module transmits the audio signal to the operation module to be transmitted thereby directly or after pre-processing to the communication device to be transmitted thereby to the terminal equipment. In this case, the audio signal is indirectly transmitted to the terminal equipment sequentially through the operation module and the communication module. And it may be arranged so that the audio signal is pre-processed by the operation module, such as analog to digital conversion, noise reduction, signal amplification and filtering.

Further, the operation module determines the blood pressure values with the auscultatory method according to the pressure signal and the audio signal or/and with the oscillometric method according to the pressure signal, and transmits the blood pressure values determined with the auscultatory method or/and the oscillometric method to the communication module to be transmitted thereby to the terminal equipment.

Further, the parameters used in the oscillometric method are adjusted on the basis of the blood pressure values determined by the operation module with the auscultatory method.

Further, the adjustment is periodic, which may be a period based on time, also may be a period based on the number of measurements or other forms.

Further, the operation module stores a plurality of sets of parameters used in the oscillometric method, so that the corresponding parameters may be selected for different users.

Further, the communication module and the audio collection module are wiredly and wirelessly connected with the terminal.

The present invention also provides a blood pressure measuring equipment, which includes:
a pressuring device used for pressurizing and depressurizing a measured site;
a sensing module used for connecting with the pressuring device to sense the pressure at the joint and output a pressure signal;
an audio collection module used for collecting the sound at the measured site and outputting an audio signal;
a master unit used for connecting with the sensing module and the audio collection module to receive and process the pressure signal and the audio signal. The processing of the pressure signal and the audio signal by the master unit includes synchronous storage for the received signals, and analog to digital conversion, noise reduction, signal amplification and filtering for the signals themselves, and data extraction, data analysis, data operation and output control based on the signals. The stored pressure signal and audio signal provide the initial data as the reference for determination with the auscultatory method and the oscillometric method, and can be used repeatedly, which provides a necessary condition for multiple determinations.

Further, the pressuring device includes a pressure-applying component directly acting on the measured site, and the audio collection module is partially or completely disposed within or outside of the pressure-applying component.

Further, the sensing module includes a pressure sensor via which the pressure at the joint between it and the pressuring device is sensed.

Further, the blood pressure measuring equipment also includes an image output module for numerically and/or graphically displaying the pressure signal and/or the audio signal. The numeralization and graphicalization of the pressure signal includes converting into corresponding pressure values and generating a corresponding trend graph, the numeralization and graphicalization of the audio signal includes extracting the values of audio amplitude, frequency and the like of the audio signal or the processed audio signal and generating a corresponding trend graph.

Further, the blood pressure measuring equipment also includes an audio output module used for playing the audio signal or the audio formed by processing of the audio signal (e.g., the audio signal has been amplified and de-noised, or has been coded or transcoded for adapting to a particular audio format). It is achieved that the sound is played and the pressure signal and the audio signal after graphicalization are shown synchronously on the blood pressure measuring equipment, so that the image also may be combined to make the determination when using the auscultatory method to determine the blood pressure values, and may be played back for multiple determinations.

Further, the master unit determines the blood pressure values with the auscultatory method and/or the oscillometric method, and the blood pressure values are displayed by the image output module or/and are played by the audio output module.

Further, the parameters used in the oscillometric method can be adjusted.

Further, the parameters used in the oscillometric method are adjusted on the basis of the values entered artificially or the blood pressure values determined by the master module with the auscultatory method.

Further, a plurality of sets of parameters used in the oscillometric method may be stored, so that the corresponding parameters may be selected for different users.

Further, when the parameters used in the oscillometric method are adjusted on the basis of the blood pressure values determined by the master module with the auscultatory method, the adjustment is periodic, which may be a period based on time, also may be a period based on the number of measurements or other forms.

Further, the master unit also includes a communication module used for wiredly or wirelessly communicating with an external equipment, for example, uploading the stored data to the external equipment such as computer, server and cloud, so that the determination for the initial data may be extended to different places and many people.

The present invention also provides a design method for blood pressure measuring equipment, the design concept of which is that, a sensing module, an audio collection module, and a master unit connected with the sensing module and the audio collection module are provided; the sensing module is used for sensing the pressure at a measured site and outputting a pressure signal; the audio collection module is used for collecting the sound at the measured site and outputting an audio signal; the master unit receives and processes the pressure signal and the audio signal. The processing of the pressure signal and the audio signal by the master unit includes synchronous storage for the received signals, and analog to digital conversion, noise reduction, signal amplification and filtering for the signals themselves, and data extraction, data analysis, data operation and output control based on the signals. The stored pressure signal and audio signal provide the initial data as the reference for determination with the auscultatory method and the oscillometric method, and can be used repeatedly, which provides a necessary condition for multiple determinations.

Further, the image output module is provided for numerically and/or graphically displaying the pressure signal and/or the audio signal. The numeralization and graphicalization of the pressure signal includes converting into corresponding pressure values and generating a corresponding trend graph, the numeralization and graphicalization of the audio signal includes extracting the values of audio amplitude, frequency and the like of the audio signal or the processed audio signal and generating a corresponding trend graph.

Further, the audio output module is provided for playing the audio signal or the audio formed by processing of the audio signal (e.g., the audio signal has been amplified and de-noised, or has been coded or transcoded for adapting to a particular audio format). It is achieved that the sound is played and the pressure signal and the audio signal after graphicalization are shown synchronously on the blood pressure measuring equipment, so that the image also may be combined to make the determination when using the auscultatory method to determine the blood pressure values, and may be played back for multiple determinations.

Further, the follow modes are employed to determine the blood pressure values:
Mode 1, the blood pressure values are determined by the master unit with the auscultatory method according to the pressure signal and the audio signal;
Mode 2, the blood pressure values are determined artificially with the auscultatory method according to the image outputted by the image output module obtained based on the pressure signal and the audio signal and the sound outputted by the audio output module;
Mode 3, the blood pressure values may be determined by the master unit with the oscillometric method according to the pressure signal, and the determined blood pressure values can be obtained immediately, where the parameters used in the oscillometric method may be adjusted to adapt to different individual circumstances.

Further, in Mode 1 and 2, the audio signal is processed by the master unit before the blood pressure values are determined with the auscultatory method, thereby further improving the accuracy of the auscultatory method.

Further, the blood pressure measuring equipment includes a plurality of sets of parameters used in the oscillometric method.

Further, in Mode 3, the parameters used in the oscillometric method are adjusted on the basis of the values entered artificially or the blood pressure values determined by the master unit with the auscultatory method, to improve the accuracy of the oscillometric method.

Further, when the parameters used in the oscillometric method are adjusted on the basis of the blood pressure values determined by the master module with the auscultatory method, the adjustment is periodic, which may be a period based on time, also may be a period based on the number of measurements or other forms.

Further, the communication module is provided for wiredly or wirelessly communicating with an external equipment, e.g., uploading the stored data to the external equipment such as computer, server and cloud, so that the determination for the initial data is extended to different places and many people.

The blood pressure measuring auxiliary device of the present invention has the following advantages:
1. simple structure, convenient connection;
2. it addresses the problem that the user cannot obtain the initial data when making a measurement using the existing sphygmomanometer products;
3. it provides a necessary data resource for storage and analysis of the initial data and visually determination according to the initial data;
4. strong adaptability, the intelligent equipment in our daily life such as mobile phone, computer, tablet computer, or new wearable equipment such as bracelet and wrist watch may be directly used as a terminal equipment;
5. it enables the existing sphygmomanometer products to be connected with the intelligent equipment and to transmit data to it, providing a necessary premise for its function expansion.

The blood pressure measuring equipment and the design method therefor of the present invention have the following advantages:
1. measurement before judgement, where the initial data may be played back synchronously, and the blood pressure is determined artificially.
   The existing sphygmomanometers calculates (determines) the blood pressure values during the measurement, either artificially, automatically or semi-automatically, and tells the blood pressure values directly to the tested person. The measurement of the initial data is separated from the determination and calculation of the blood pressure values with the products and methods of the present invention, the initial data is recorded during the measurement, and the initial data is analyzed in various ways after the measurement to obtain the blood pressure values. The stored initial data may be analyzed repeatedly by different devices and different people, so as to obtain more accurate blood pressure values. By taking the traditional auscultatory method as an example, it is relatively difficult to determine the sound while keeping the eyes on the pressure gage during pressurization and depressurization of cuffed air bladder. However, in the products and methods of the present invention, the initial data can be listened to repeatedly, can be listened to by many people, and can be listened to by a professional person, after it is recorded; the volume can be tuned up when the sampled sound is small, and noise reduction processing can be firstly performed for it when the noise in the sampled sound is too big.
2. The visualization of the initial data, especially the visualization of the sound facilitates determining the blood pressure with the auscultatory method.

Determination with the traditional auscultatory method or determination directly by listening to the sound, or determination by machine according to the sound are all made during pressurizing and depressurizing by the pressuring device. In the present invention, sound is visualized, and can be synchronously shown when the sound is played back, which has the effect of assisting in determining the sound, reduces the difficulty in the artificial auscultatory method, and improves the accuracy of the automatic auscultatory method. The visualization of the sound allows people to determine the sound from only using ears to using ears and eyes, which reduces the difficulty of determining the beating sound of blood vessels. Visualized sound can also allow people to make a predetermination before a certain sound is played, for example, the intensity amplitude or frequency of the sound next second can be seen when sound is played to a certain position.
3. The parameters used in the oscillometric method, which are custom-calibrated and dynamically calibrated according to the personal circumstances, are varied from person to person, thereby improving the accuracy.

In the existing sphygmomanometers, the parameters used in the oscillometric method are fixed, or are simple packets. With the products and methods of the present invention, each individual owns the parameters set to suit for themselves according to correction, thereby improving the accuracy of the oscillometric method.

### Brief Description of the Drawings

Figure 1 is a graph of the frequency distribution of measurement deviations of a certain brand sphygmomanometer;
Figure 2 is a graph of the curve coordinates of normalization values of the amplitude parameter method;
Figure 3 is a schematic diagram of a blood pressure measuring auxiliary device of the present invention, with the sphygmomanometer and the mobile phone that may be used with it being shown;
Figure 4 is a schematic diagram of the structure of an access component of the blood pressure measuring auxiliary device shown in Figure 3;
Figure 5 is a schematic diagram of connections between the blood pressure measuring auxiliary device shown in Figure 3, the sphygmomanometer and the mobile phone;
Figure 6 is a schematic diagram of a sphygmomanometer according to the present invention;
Figure 7 is a schematic diagram of a host system architecture of the sphygmomanometer shown in Figure 6;
Figure 8 is a schematic diagram of an operator interface of the sphygmomanometer shown in Figure 6; and
Figure 9 is another schematic diagram of the operator interface shown in Figure 8.

### Detailed Description of the Preferred Embodiments

The blood pressure measuring auxiliary device and the blood pressure measuring equipment of the present invention is further illustrated below by the specific embodiments, in order to fully appreciate the objects, features, design methods and effects of the present invention.

### Embodiment 1

This embodiment is a measuring auxiliary device for blood pressure measurement according to the present invention, which mainly solves the problem that the user cannot obtain the initial data when making the measurement using the existing sphygmomanometer products. An upper arm type electronic sphygmomanometer is used as an example in this embodiment, but it is not limited to the upper arm type electronic sphygmomanometer. Other sphygmomanometers with different pressuring manners and for different measured sites such as mercury sphygmomanometer and wrist electronic sphygmomanometer may also be used, as long as a pressuring device is provided in the used sphygmomanometer.

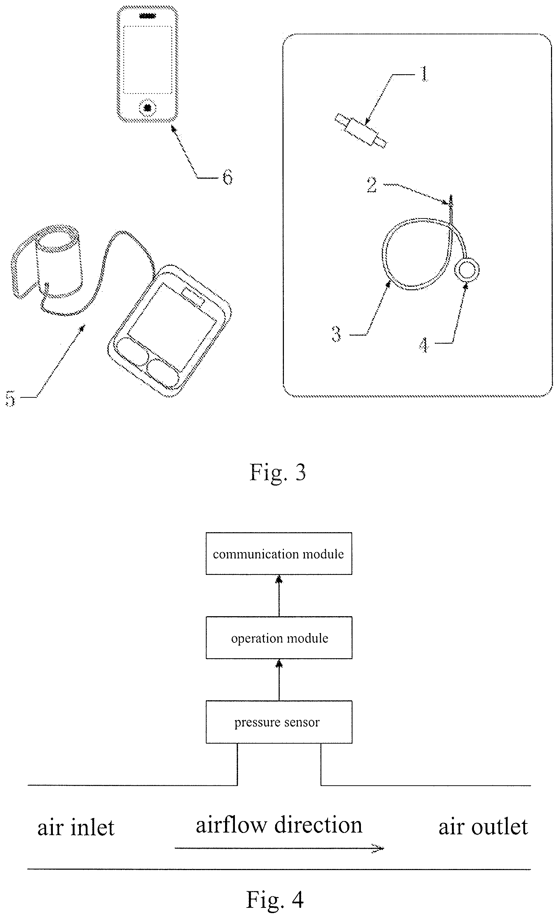

As shown in Figure 3, within the solid box is a measuring auxiliary device according to the present invention, which is used in conjunction with an upper arm type electronic sphygmomanometer 5, wherein the measuring auxiliary device includes an access component 1, a microphone 2, an air tube 3 and a stethoscope 4. The microphone 2 is connected with the stethoscope 4 through the air tube 3, the microphone 2 is provided at one end with a headset plug connected with the mobile phone 6, so as to form an audio collection module. In this embodiment, the mobile phone 6 is used as a terminal equipment, but it is not limited to the use of the mobile phone, intelligent equipment in our daily life such as computer and a tablet computer or new wearable equipment such as bracelet and wrist watch also may be used.

As shown in Figure 4, the access component 1 includes an operation module (such as a single-chip microcomputer), an air inlet and an air outlet, a communication module and a pressure sensor; the air inlet, the air outlet and the port connected with the pressure sensor in the access component 1 is a three-way structure. In this embodiment, the communication module is wirelessly connected with the mobile phone 6 to achieve data transmission. A wired connection also can be employed, and a data cable is arranged to be connected to the data port of the mobile phone 6, such as USB port.

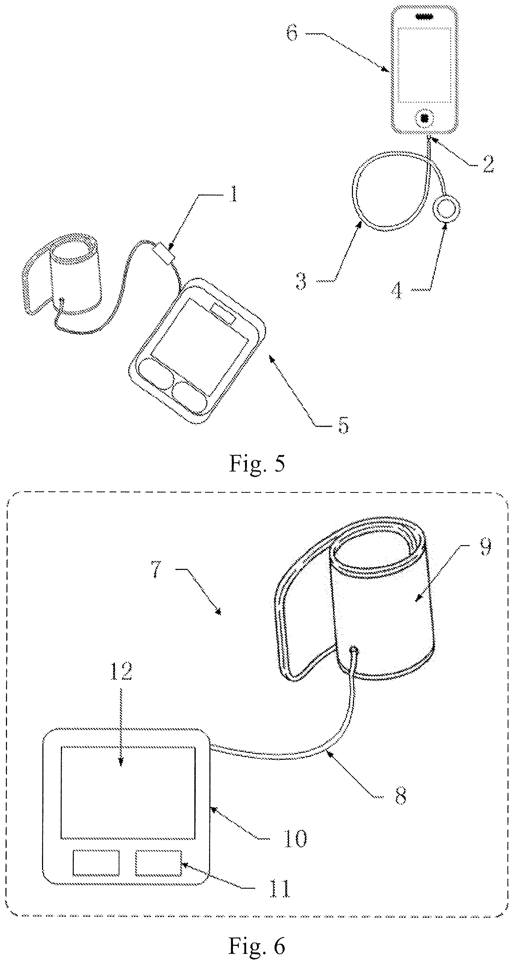

When people need to make the blood pressure measurement, as shown in Figure 5, the access component 1 is connected into the air tube of the upper arm type electronic sphygmomanometer 5, and the headset plug at one end of the microphone 2 is inserted into the headset jack of the mobile phone 6. The upper arm type electronic sphygmomanometer 5 is used in its normal use mode, the cuff is bundled at the brachial artery of upper arm, and simultaneously the stethoscope 4 needs to be placed inside the cuff close to the brachial artery. The measurement procedure of the upper arm type electronic sphygmomanometer 5 is performed; the measured site is pressurized and depressurized. During this process, the pressure sensor of the access component 1 will sense the pressure in the air tube and output a pressure signal, after the pressure signal is converted, amplified and filtered via the operation module (the operation module may not be used, in this case the output signal of the pressure sensor is directly transmitted to the mobile phone 6 by the communication module), the pre-processed pressure signal is transmitted to the mobile phone 6 through the communication module, at the same time the microphone 2 collects the sound at the brachial artery that is transmitted via the stethoscope 4 and the air tube 3, and which is transmitted to the mobile phone 6 by the headset plug. Similar to the pre-processing of the pressure signal, a conversion module for pre-processing the audio signal may also be added at the output end of the microphone 2, such as performing the processing like signal amplification, noise reduction, digital-to-analogue conversion and filtering, and the output from the headset plug is the pre-processed audio signal.

Thus, the collection and transmission of the pressure and audio signals at the measured site during the measurement made by the existing sphygmomanometer products are achieved with the measuring auxiliary device.

On the other hand, the components within the box of Figure 3 may also be combined in several different ways, for example, the microphone 2 may be placed in the stethoscope 4, and then is connected to the headset plug by the conductive wire, in this case, there is no need to use the air tube 3 or other sound conducting medias. The connection between the headset plug and the mobile phone 6 may be a direct connection, or it may be an indirect connection, for example, the headset jack is arranged on the access component 1, the headset plug is inserted into the access component 1; the audio signal is transmitted via the wired or wireless connection between the access component 1 and the mobile phone 6, there are also two ways in this case: (1) the audio signal is directly transmitted to the mobile phone 6 by the communication module; (2) since the pressure signal and the audio signal can be received simultaneously by the access component 1, the pre-processing of the audio signal can be realized in the operation module (such as the conversion module described above), then the pre-processed audio signal is transmitted to the communication module which then transmits it to the mobile phone 6.

While the functions of the operation module can be extended as needed, to mitigate the data processing pressure of the terminal equipment, even only using the terminal equipment as the output equipment of image and sound, the processing of data can be performed by the operation module, such as the described above pre-processing of the pressure signal and the audio signal, and the blood pressure values are also determined with the oscillometric method or the auscultatory method according to the received signals, and then transmitted to the terminal equipment through the communication module.

Therefore, most of the functions (such as data sync storage, data processing and calculating, determination with the oscillometric method, the parameter storage and adjustment with the oscillometric method, etc.) that are realized by a specialized APP on the mobile phone 6 all can be achieved by arranging corresponding function modules in the operation module, the mobile phone 6 is responsible only for displaying and man-machine interaction.

The data therein may also be transmitted to the external equipment via the wired or wireless connection with the mobile phone 6, for example, the stored data is uploaded to the external equipment such as computer, server and cloud, and is shared on the external equipment or is directly played back on the external equipment, so that the determination of blood pressure values is no longer limited by time, the number of people and the physical distance.

### Embodiment 2

This embodiment is an electronic sphygmomanometer according to the present invention, which employed pressure control, signal/data collection, signal/data processing, signal/data analysis, signal/data watching and listening to and man-machine interaction.

As shown in Figure 6, which is an upper arm type electronic sphygmomanometer 7 of this embodiment, but it is not limited to the upper arm type electronic sphygmomanometer, other types of sphygmomanometers with different pressuring manners or against different measured sites are possible. The sphygmomanometer includes a host 10, an air line 8 and a cuffed air bladder 9, and the host 10 includes a button 11 and a touch display screen 12. A stethoscope (not shown) is fixed inside the cuffed air bladder 9, a microphone (not shown) is provided in the cavity of the stethoscope (it is possible to only use the microphone, and the stethoscope is not provided). The microphone is connected to the conductive wire of the host 10 and embedded in the air line 8, so that it has little difference from a common upper arm type sphygmomanometer in appearance.

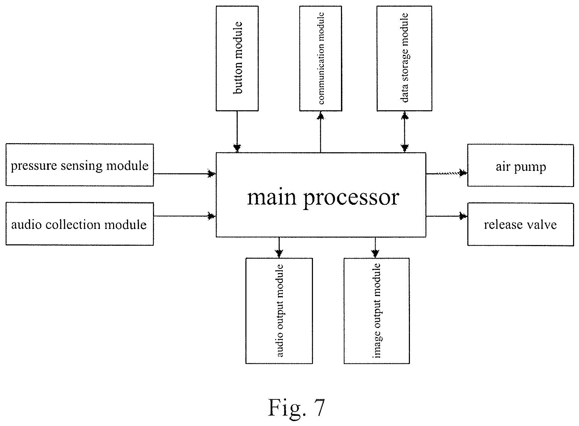

As shown in Figure 7, which is a system architecture of the host 10, where the main processor is mainly responsible for: controlling the air pump and the release valve in the pressuring device; processing the pressure signal and the audio signal transmitted by the pressure sensing module and the audio collection module respectively (such as amplification, noise reduction, analog to digital conversion and filtering) and synchronously storing to the data storage module; determining the blood pressure values with the oscillometric method and/or the auscultatory method; controlling the output of audio and image; receiving instructions from the button module to perform operations such as device control, parameter adjustment, data playback. The button input from the user (the receiving end of the button module) may be achieved by a physical button, or it may be achieved by touching the touch display screen, a combination of the two ways is used in this embodiment.

The pressure sensing module senses the pressure via the pressure sensor, which may send the pressure signal outputted by the pressure sensor directly to the main processor for processing, or an operation module as described in Embodiment 1 is arranged within the pressure sensing module for pre-processing the pressure signal.

A communication module is also included in the architecture, which is used for wired or/and wireless communication between the host 10 and the external equipment, the communication module in this embodiment uses a wireless protocol for data transmission and sharing with the external equipment.

The operator interface of the sphygmomanometer in this embodiment is further illustrated below, it is to be noted that the setting of the interface is not limited to the following example, numerous changes may be made on the basis of the prior art. The following example is intended to reflect the beneficial effects of the visualization of initial data on blood pressure measurement and determination.

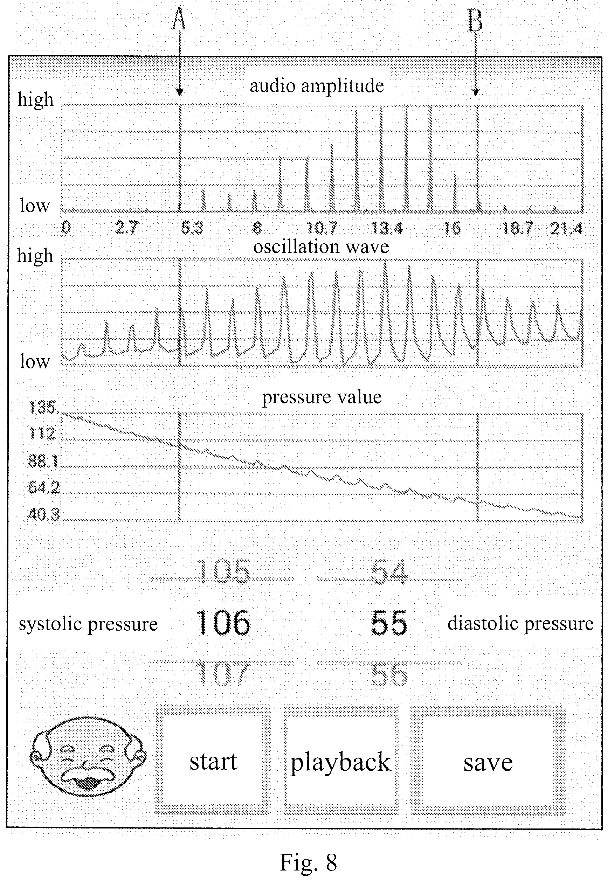

As shown in Figure 8, which is an operator interface of the upper arm type electronic sphygmomanometer 7, where graphs of audio amplitude, oscillation wave and pressure value over time, and blood pressure values calculated by the main processor according to the auscultatory method (the blood pressure values may also be provided directly by voice after the measurement) are shown from top to bottom, respectively. The blood pressure values determined with the oscillometric method may also be shown, or the blood pressure values determined with the auscultatory method and the oscillometric method are shown simultaneously. In each of the graphs, the leftmost vertical line A denotes the time point corresponding to the systolic pressure determined according to the auscultatory method, and the rightmost vertical line B denotes the time point corresponding to the diastolic pressure.

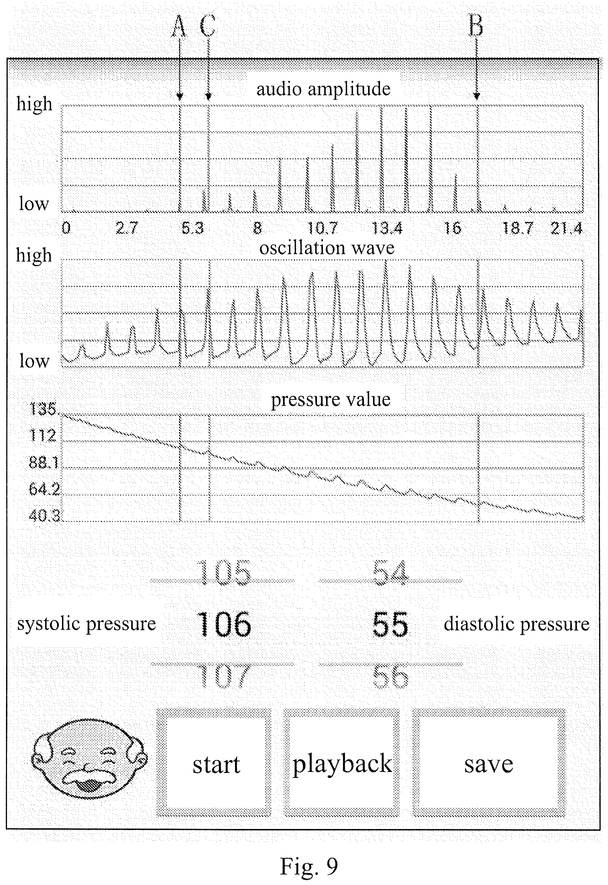

Figure 9 is the state of the interface in Figure 8 when the data is played back. As the audio is played, the vertical line C at the middle in the operator interface could move along the time axis with the playing progress, the audio amplitude curve that the vertical line C passes through may help the user to more accurately determine the sound that the ears heard. In addition, the graphicalization of the audio signal not only may be in the time domain, but also in the frequency domain or both. The graphs are also not limited to be curve graphs, or may be column graphs etc.. It is also possible to suspend, continue in the playback process of sound and to arrange a loop play between two time points.

For the blood pressure values determined by the main processor with the oscillometric method, they can also be calibrated according to the blood pressure values obtained with the auscultatory method (or the blood pressure values with higher accuracy obtained by other ways), by taking the amplitude parameter method in the oscillometric method as an example, the parameters C1 and C2 vary from person to person, which can be adjusted according to personal circumstances, to improve the accuracy of measurement for individuals. The adjustment can modify the parameters used in the oscillometric method directly by the operator interface (not shown), such as the parameter C1 and C2, but this may need professional person to be implemented effectively. For general users, it may be arranged so that, for a certain measurement, the blood pressure values that are considered to be accurate after confirmation (such as the blood pressure values determined artificially with the auscultatory method) are inputted, the inputted blood pressure valves are compared with the blood pressure values determined with the oscillometric method by the specialized APP, the parameter adjustment is carried out according to the comparative result, so that the parameter adjustment may also be simply carried out without the need for professional person. The parameters used in the oscillometric method (such as C1 and C2 in the amplitude parameter method) are automatically calibrated on the basis of the blood pressure values determined with the auscultatory method, the calibration may also be arranged to be periodic, for example, it is arranged so that they are automatically calibrated after a period of time or a certain number of times.

Since the modification of parameters is the calibration that is carried out for personal circumstances, the accuracy of measurement will be generally higher than the direct reading on an ordinary electronic sphygmomanometer. And after calibration, even without using the stethoscope 4 (to determine blood pressure values without using the auscultatory method), it is also possible to obtain relatively accurate blood pressure values. The avatar icon in the lower left of the operator interface in Figures 6 and 7 can be used for switching users, each user has a respective parameter group, and many people use is satisfied.

The implement of the above interface functions are all within the achievable scope of prior art, and as long as there is a data source, the mode of processing and displaying data is varied, such as correlation analysis is carried out for the data, dynamic elements are added into the image, etc., further design or improvement can be done according to actual needs.

In use, as an ordinary electronic sphygmomanometer, the sphygmomanometer of this embodiment is started just by pushing a button, and the sphygmomanometer automatically inflates or deflates the cuffed air bladder, and records the measured data simultaneously. Once the measurement is complete, the blood pressure values determined with the oscillometric method and/or the auscultatory method are displayed automatically, the recorded pressure values and sound data may also be played back on the host, and the blood pressure values are determined artificially with the auscultatory method.

Finally, it should be pointed out that, in Embodiment 1, almost all functions of the sphygmomanometer in this embodiment may be achieved by programming a corresponding APP on the mobile phone (or computer, tablet computer and the like) as the terminal equipment. In this case, the mobile phone is roughly comparable to the host 10 in this embodiment, and the difference is that the mobile phone cannot control the pressuring device.

The preferred specific embodiments of the invention have been described in detail above. It is to be understood that numerous modifications and variations can be made by those ordinary skilled in the art in accordance with the concepts of the present invention without any inventive effort. Hence, the technical solutions that may be derived by those skilled in the art according to the concepts of the present invention on the basis of the prior art through logical analysis, reasoning and limited experiments should be within the scope of protection defined by the claims.

## Claims

1. A measuring auxiliary device for blood pressure measurement, wherein the measuring auxiliary device is arranged to be connected with a pressuring device of a sphygmomanometer, and comprises a sensing module and a communication module;
the sensing module is used for sensing the pressure at the joint between the measuring auxiliary device and the pressuring device and outputting a pressure signal to the communication module;
the communication module is used for receiving the pressure signal which is then transmitted to a terminal equipment.

2. The measuring auxiliary device according to claim 1, wherein the sensing module comprises a pressure sensor via which the pressure at the joint between the measuring auxiliary device and the pressuring device is sensed.

3. The measuring auxiliary device according to claim 1, further comprising an operation module used for pre-processing the pressure signal and transmitting the pre-processed pressure signal to the communication module to be transmitted thereby to the terminal equipment.

4. The measuring auxiliary device according to claim 3, wherein the operation module determines the blood pressure values by the oscillometric method according to the pressure signal and transmits the blood pressure values to the communication module to be transmitted thereby to the terminal equipment.

5. The measuring auxiliary device according to claim 1, wherein the communication module is wiredly or wirelessly connected with the terminal.

6. A measuring auxiliary device for blood pressure measurement, wherein the measuring auxiliary device is arranged to be connectable with a pressuring device of a sphygmomanometer, and comprises a sensing module, a communication module and an audio collection module;
the sensing module is used for sensing the pressure at the joint between the measuring auxiliary device and the pressuring device and outputting a pressure signal to the communication module;
the communication module is used for receiving the pressure signal and transmitting the pressure signal to a terminal equipment;
the audio collection module is used for collecting the sound at the measured site and outputting an audio signal to the terminal equipment.

7. The measuring auxiliary device according to claim 6, wherein the sensing module comprises a pressure sensor via which the pressure at the joint between the measuring auxiliary device and the pressuring device is sensed.

8. The measuring auxiliary device according to claim 7, further comprising an operation module for pre-processing the pressure signal and transmitting the pre-processed pressure signal to the communication module to be transmitted thereby to the terminal equipment.

9. The measuring auxiliary device according to claim 8, wherein the operation module determines the blood pressure values by the oscillometric method according to the pressure signal and transmits the blood pressure values to the communication module to be transmitted thereby to the terminal equipment.

10. The measuring auxiliary device according to claim 6, wherein the audio collection module comprises a conversion module to pre-process the collected sound.

11. The measuring auxiliary device according to claim 6, wherein the audio collection module transmits the audio signal to the communication module to be transmitted thereby to the terminal equipment.

12. The measuring auxiliary device according to claim 8, wherein the audio collection module transmits the audio signal to the operation module to be transmitted thereby directly or after pre-processing to the communication module, then to be transmitted thereby to the terminal equipment.

13. The measuring auxiliary device according to claim 12, wherein the operation module determines the blood pressure values with the auscultatory method according to the pressure signal and the audio signal or/and with the oscillometric method according to the pressure signal, and transmits the blood pressure values determined with the auscultatory method or/and the oscillometric method to the communication module to be transmitted thereby to the terminal equipment.

14. The measuring auxiliary device according to claim 13, wherein the parameters used in oscillometric method are adjusted on the basis of the blood pressure values determined by the operation module with the auscultatory method.

15. The measuring auxiliary device according to claim 14, wherein the adjustment is periodic.

16. The measuring auxiliary device according to claim 14, wherein the operation module stores a plurality of sets of parameters used in the oscillometric method.

17. The measuring auxiliary device according to claim 6, wherein the communication module and the audio collection module are wiredly or wirelessly connected with the terminal.

18. A blood pressure measuring equipment, comprising
a pressuring device used for pressurizing and depressurizing a measured site;
a sensing module used for connecting with the pressuring device to sense the pressure at the joint and output a pressure signal;
an audio collection module used for collecting the sound at the measured site and outputting an audio signal;
a master unit used for connecting with the sensing module and the audio collection module to receive and process the pressure signal and the audio signal.

19. The blood pressure measuring equipment according to claim 18, wherein the pressuring device comprises a pressure-applying component directly acting on the measured site, and the audio collection module is partially or completely disposed within or outside of the pressure-applying component.

20. The blood pressure measuring equipment according to claim 18, wherein the sensing module comprises a pressure sensor via which the pressure at the joint between the pressure sensor and the pressuring device is sensed.

21. The blood pressure measuring equipment according to claim 18, further comprising an image output module for numerically and/or graphically displaying the pressure signal and/or the audio signal.

22. The blood pressure measuring equipment according to claim 21, further comprising an audio output module used for playing the audio signal and the audio formed by processing of the audio signal.

23. The blood pressure measuring equipment according to any of claims 18-22, wherein the master unit determines the blood pressure values with the auscultatory method and/or the oscillometric method, and the blood pressure values are displayed by the image output module or/and are played by the audio output module.

24. The blood pressure measuring equipment according to claim 23, wherein the parameters used in the oscillometric method can be adjusted.

25. The blood pressure measuring equipment according to claim 24, wherein the parameters used in the oscillometric method are adjusted on the basis of the values entered artificially or the blood pressure values determined by the master unit with the auscultatory method.

26. The blood pressure measuring equipment according to claim 24, wherein a plurality of sets of parameters used in the oscillometric method are stored.

27. The blood pressure measuring equipment according to claim 25, wherein when the parameters used in the oscillometric method are adjusted on the basis of the blood pressure values determined by the master unit with the auscultatory method, the adjustment is periodic.

28. The blood pressure measuring equipment according to claim 18, wherein the master unit further comprises a communication module used for wiredly or wirelessly communicating with an external equipment.

29. A design method for blood pressure measuring equipment, wherein a sensing module, an audio collection module, and a master unit connected with the sensing module and the audio collection module are provided; the sensing module is used for sensing the pressure at a measured site and outputting a pressure signal; the audio collection module is used for collecting the sound at the measured site and outputting an audio signal; the master unit receives and processes the pressure signal and the audio signal.

30. The design method according to claim 29, wherein an image output module is provided for numerically and/or graphically displaying the pressure signal and/or the audio signal.

31. The design method according to claim 30, wherein an audio output module is provided for playing the audio signal or the audio formed by processing of the audio signal.

32. The design method according to any of claims 29-31, wherein the following modes are employed to determine the blood pressure values:
Mode 1, the blood pressure values are determined by the master unit with the auscultatory method according to the pressure signal and the audio signal;
Mode 2, the blood pressure values are determined artificially with the auscultatory method according to the image outputted by the image output module obtained based on the pressure signal and the audio signal and the sound outputted by the audio output module;
Mode 3, the blood pressure values are determined by the master unit with the oscillometric method according to the pressure signal, wherein the parameters used in the oscillometric method can be adjusted.

33. The design method according to claim 32, wherein in Mode 1 and 2, the audio signal is processed by the master unit before the blood pressure values are determined with the auscultatory method.

34. The design method according to claim 32, wherein the blood pressure measuring equipment comprises a plurality of sets of parameters used in the oscillometric method.

35. The design method according to claim 32, wherein in Mode 3, the parameters used in the oscillometric method are adjusted on the basis of the values entered artificially or the blood pressure values determined by the master unit with the auscultatory method.

36. The design method according to claim 35, wherein when the parameters used in the oscillometric method are adjusted on the basis of the blood pressure values determined by the master unit with the auscultatory method, the adjustment is periodic.

37. The design method according to claim 29, wherein a communication module is provided for wiredly or wirelessly communicating with an external equipment.
